# EUROPEAN PATENT APPLICATION

(11) **EP 2 540 821 A1**
(43) Date of publication of application: **02.01.2013**
(21) Application number: 11747355.3
(22) Date of filing: 23.02.2011
(51) Int. Cl.: C12N 5/0784, A61K 35/12, A61P 29/00, A61P 31/04, A61P 37/06, C07D 487/04

(54) **METHOD FOR PRODUCING REGULATORY DENDRITIC CELLS**

(30) Priority: 23.02.2010 JP 2010037039
(71) Applicant: Nippon Kayaku Kabushiki Kaisha, Tokyo 102-8172 (JP); National University Corporation Hokkaido University, Sapporo-shi, Hokkaido 060-0808 (JP)
(72) Inventor: TODO, Satoru, Sapporo-shi Hokkaido 060-0808 (JP); YAMASHITA, Kenichiro, Sapporo-shi Hokkaido 060-0808 (JP); SHIBASAKI, Susumu, Sapporo-shi Hokkaido 060-0808 (JP)
(74) Representative: Polypatent
(86) International application number: PCT/JP2011/053906
(87) International publication number: WO 2011/105394

(57) **Abstract**

An object of the present invention is to establish a method that enables safe and convenient large-scale preparation of regulatory dendritic cells. The present invention provides a method for preparing regulatory dendritic cells, which comprises culturing cells that can be induced to result in regulatory dendritic cells in the presence of a [1,2,4]triazolo[1,5-a]pyrimidine derivative.

## Description

### Technical Field

The present invention relates to a method for preparing regulatory dendritic cells to be used for preventing and treating diseases such as transplantation rejections, graft-versus-host disease, autoimmune disease, allergic disease, chronic inflammatory disorder, and septicemia which results from abnormal responses and excessive responses of the immune system. The present invention more specifically relates to a method for preparing regulatory dendritic cells using a [1,2,4]triazolo[1,5-a]pyrimidine derivative, and particularly (S)-(+)-1-(5-hydroxy-1,5-dimethylhexyl)-3-[7-(4-methoxyphenyl)-[1,2,4]triazole[1,5-a]pyrimidin-2-yl]urea (NK026680). Furthermore, the present invention relates to regulatory dendritic cells prepared by the above method and a pharmaceutical composition or a pharmaceutical kit comprising the aforementioned regulatory dendritic cells. Furthermore, the present invention relates to the use of a [1,2,4]triazolo[1,5-a]pyrimidine derivative for preparing regulatory dendritic cells.

### Background Art

Diseases resulting from immunological abnormalities and excessive immune responses have been mainly prevented and treated using medicaments such as immunosuppressants, anti-inflammatory agents, and antiallergic agents. Although treatment with such medicaments is effective, it is problematic in that it lowers patients' QOL due to adverse effect.

These medicaments are also transferred to tissues other than target immune cells. A drug exhibits non-specific effects in such non-targeted tissues, so that adverse effects take place. Such adverse effects often force administration to be halted even when immunosuppressive effects have been exhibited. Alternatively, when medical procedures or the like are performed to alleviate an adverse effect, the patient's QOL may be lowered. Although it has been attempted to alleviate adverse effects through local administration, this is applicable only for atopic dermatitis, bronchial asthma, and the like wherein the affected parts are localized in specific tissues or organs.

Hence, prevention and treatment of the above diseases using regulatory dendritic cells have been attempted. Dendritic cells are antigen presenting cells having dendrites, and they widely exist as immature dendritic cells in peripheral non-lymphatic tissues and lymphatic tissues. In inflammatory tissue invaded by microorganisms, viruses, and foreign matter, dendritic cells phagocytize the foreign antigens so that they differentiate into mature dendritic cells and then migrate to secondary regional lymphatic tissues. Here, mature dendritic cells activate native T cells through a two signal mechanism, which consists of an antigen-specific signal and a co-stimulatory signal to induce differentiation thereof into antigen-specific effector T cells, thereby inducing immune responses. As described above, dendritic cells are strong antigen presenting cells bridging natural immunity and acquired immunity.

Meanwhile, in an inflammatory environment, some dendritic cells express lower levels of cosfimulatory molecules. It has been suggested that these dendritic cells regulate T cells in a suppressive manner so as to induce immunologic tolerance to the body's own tissues, self antigens, or the like. Such dendritic cells are referred to as regulatory dendritic cells. Immunocyte therapies using regulatory dendritic cells have exhibited therapeutic effects through induction of antigen-specific T cell unresponsiveness or regulatory T cell development in the model mice of graft -versus-host disease, autoimmune disease or allergic disease (Non-patent Documents 1, 2, and 3). Furthermore, in a mouse septicemia model, regulatory dendritic cells have inhibited the production of inflammatory cytokines through IL-10 production, showing a survival effect (Non-patent Document 4).

Patent Document 1 discloses a method for inducing human immunoregulatory dendritic cells by culturing human dendritic cells or precursor cells thereof *in vitro* with cytokines including at least IL-10 and TGF-β, and human immunoregulatory dendritic cells obtained by the method. Patent Document 2 discloses an attempt to use a regulatory dendritic cell as an agent for promoting IL-10 production for systemic inflammatory response syndrome.

Clinical application of the above-described immunocyte therapies using regulatory dendritic cells for immune disease has been attempted. Actual clinical application requires the establishment of a large-scale preparation method in addition to the achievement of the safety of regulatory dendritic cells. As disclosed in Non-patent Document 1, cytokines including IL-10 and TGF-β are used for the preparation of regulatory dendritic cells which have previously been reported. These cytokines are produced from gene recombinants of *Escherichia coli* and the like, so that safety issues and production costs that interfere with large-scale preparation remain.

Moreover, a [1,2,4]triazolo[1,5-a]pyrimidine derivative (Patent Documents 3 and 4) is a substance inhibiting the functions of dendritic cells, and it exhibits suppressive effects on mouse delayed hypersensitivity reaction (Patent Document 4). One of such derivatives, (S)-(+)-1-(5-hydroxy-1,5-dimethylhexyl)-3-[7-(4-methoxyphenyl)-[1,2,4]triazolo[1,5-a]pyrimidin-2-yl] urea (hereinafter, referred to as "NK026680"), the expression level of a costimulatory molecule decreased, and NK026680 exhibited therapeutic effects against graft-versus-host disease models and an autoimmune vasculitis model (Non-patent Documents 5 and 6).

### Prior Art Documents

### Patent documents

Patent Document 1: JP Patent Publication (Kokai) No. 2004-298181 A
Patent Document 2: JP Patent Publication (Kokai) No. 2006-290761 A
Patent Document 3: JP Patent Publication (Kokai) No. 2005-154335 A
Patent Document 4: International Patent Publication WO2004/108729
Non-patent Documents

Non-patent Document 1: Sato K, Yamashita N, Yamashita N, Baba M, Matsuyama T. Regulatory dendritic cells protect mice from murine acute graft-versus-host disease and leukemia relapse. Immunity 2003; 18(3): 367-379.
Non-patent Document 2: Sato K, Yamashita N, Baba M, Matsuyama T. Modified myeloid dendritic cells act as regulatory dendritic cells to induce anergic and regulatory T cells. Blood 2003; 101(9): 3581-3589.
Non-patent Document 3: Fujita S, Yamashita N, Ishii Y, Sato Y, Sato K, Eizumi K, Fukaya T, Nozawa R, Takamoto Y, Yamashita N, Taniguchi M, Sato K, Regulatory dendritic cells protect against allergic airway inflammation in a murine athmatic model. J Allergy Clin Immunol 2008; 121(1): 95-104.
Non-patent Document 4: Fujita S, Seino K, Sato K, Sato Y, Eizumi K, Yamashita N, Taniguchi M, Sato K. Regulatory dendritic cells act as regulators of acute lethal systemic inflammatory response. Blood 2006; 107(9): 3656-3664.
Non-patent Document 5: Saiga K, Toyoda E, Tokunaka K, Masuda A, Matsumoto S, Mashiba H, Kuramochi H, Nemoto K, Abe F, Kawagishi N, Furokawa H, Ono M. NK026680, a novel compound suppressive of dendritic cell function, ameliorates mortality in acute lethal graft-versus-host reaction in mice. Bone Marrow Transplant 2006; 37(3): 317-323.
Non-patent Document 6: Saiga K, Yoshida M, Nakamura I, Toyoda E, Tokunaka K, Morohashi H, Abe F, Nemoto K, Nose M. Evaluation of the ameliorative effects of immunosuppressants on crescentic glomerulonephritis in SCG/Kj mice. Int Immunopharmacol 2008; 8(9): 1183-1189.

### Disclosure of the Invention

### Problem to Be Solved by the Invention

An object to be solved by the present invention is to establish a method that enables safe and convenient large-scale preparation of regulatory dendritic cells. Another object to be solved by the present invention is to provide regulatory dendritic cells which are useful for preventing and treating immune system diseases while maintaining patients' QOL.

### Means for Solving the Problem

As a result of intensive studies to achieve the above objects, the present inventors have discovered that regulatory dendritic cells can be prepared by culturing regulatory dendritic cells in the presence of a [1,2,4]triazolo[1,5-a]pyrimidine derivative, and thus have completed the present invention.

The present invention provides the following invention.
(1) A method for preparing regulatory dendritic cells, which comprises culturing cells that can be induced to result in regulatory dendritic cells in the presence of a [1,2,4]triazolo [1,5-a] pyrimidine derivative.
(2) The method for preparing regulatory dendritic cells according to (1), wherein the [1,2,4]triazolo[1,5-a]pyrimidine derivative is (S)-(+)-1-(5-hydroxy-1,5-dimethylhexyl)-3-[7-(4-methoxyphenyl)-[1,2,4]triazolo[1,5-a]pyrimidine-2-il] urea.
(3) The method for preparing regulatory dendritic cells according to (1) or (2), wherein the cells that can be induced to result in regulatory dendritic cells are dendritic cells or precursor cells thereof
(4) The method for preparing regulatory dendritic cells according to (3), wherein the precursor cells of dendritic cells are monocytes.

(5) The method for preparing regulatory dendritic cells according to any one of (1) to (4), wherein the cells that can be induced to result in regulatory dendritic cells are cells derived from peripheral blood, bone marrow, spleen, or umbilical cord blood.
(6) The method for preparing regulatory dendritic cells according to any one of (1) to (5), which comprises culturing the cells in the presence of GM-CSF and IL-4.
(7) The method for preparing regulatory dendritic cells according to any one of (1) to (6), which comprises culturing the cells in the presence of INF-α and/or LPS.

(8) A regulatory dendritic cell, which is prepared by the preparation method according to any one of (1) to (7).
(9) The regulatory dendritic cell according to (8), wherein the expression levels of CD40, CD80, and CD86 are lower than those of cells obtained by culturing cells that can be induced to result in regulatory dendritic cells in the absence of a [1,2,4]triazolo[1,5-a]pyrimidine derivative.
(10) The regulatory dendritic cell according to (8) or (9), wherein the production levels of IL-6 and IL-12p40 are lower than those of cells obtained by culturing cells that can be induced to result in regulatory dendritic cells in the absence of the [1,2,4]triazolo[1,5-a]pyrimidine derivative.
(11) The regulatory dendritic cell according to any one of (8) to (10), which has a lower capacity to induce T cell activation against an alloantigen than cells obtained by culturing cells that can be induced to result in regulatory dendritic cells in the absence of the [1,2,4]triazolo[1,5-a] pyrimidine derivative.

(12) A pharmaceutical composition or a pharmaceutical kit, which comprises the regulatory dendritic cell according to any one of (8) to (11).
(13) The pharmaceutical composition or a pharmaceutical kit according to (12), which is used for preventing and/or treating transplantation rejections, graft-versus-host disease, autoimmune disease, allergic disease, inflammatory disease, septicemia or shock which results from abnormal responses and excessive responses of the immune system.

(14) Use of a [1,2,4]triazolo[1,5-a]pyrimidine derivative for preparing regulatory dendritic cells.
(15) The use according to (14), wherein the [1,2,4]triazolo[1,5-a]pyrimidine derivative is (S)-(+)-1-(5-hydroxy-1,5-dimethylhexyl)-3-[7-(4-methoxyphenyl)-[1,2,4]triazolo[1,5-a] pyrimidin-2-yl]urea.

(16) A reagent for inducing regulatory dendritic cells, which comprises a [1,2,4]triazolo[1,5-a]pyrimidine derivative.
(17) The reagent for inducing regulatory dendritic cells according to (16), wherein the [1,2,4]triazolo[1,5-a]pyrimidine derivative is (S)-(+)-1-(5-hydroxy-1,5-dimethylhexyl)-3-[7-(4-methoxyphenyl)-[1,2,4]triazolo[1,5-a]pyrimidin-2-yl]urea.

### Effects of the Invention

In the present invention, a [1,2,4]triazolo[1,5-a]pyrmidine derivative is useful for inducing regulatory dendritic cells *ex vivo* from mononuclear cells contained in peripheral blood cells, bone marrow cells, or the like. The [1,2,4]triazolo[1,5-a]pyrimidine derivative is a low-molecular-weight compound that can be produced at low cost and can ensure large-scale production and safety. Through the use of the [1,2,4]triazolo[1,5-a]pyrimidine derivative for preparing regulatory dendritic cells, a large amount of regulatory dendritic cells can be obtained safely and conveniently. According to the present invention, regulatory dendritic cells can be prepared at low cost in a large amount without the use of expensive recombinant cytokines having unstable physical properties. Furthermore, since medicaments that have been used for production do not remain in the regulatory dendritic cells of the present invention, the regulatory dendritic cells are extremely effective for preventing and treating diseases resulting from abnormalities in the immune system and excessive immunoresponses such as myelosuppression, organ transplantation, autoimmune disease, allergic disease, and shock, without causing any adverse effect due to the medicaments used for production.

### Brief Description of the Drawings

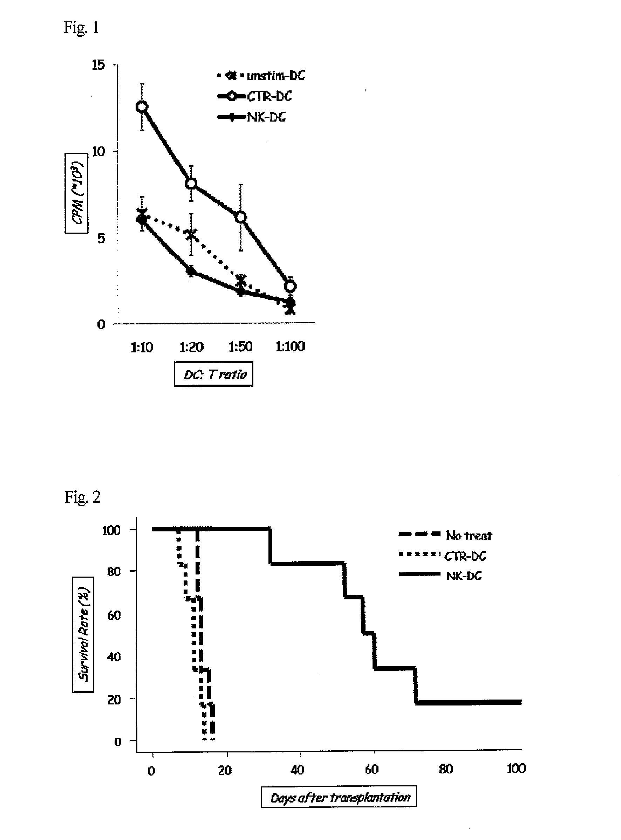

[Figure 1] Fig. 1 shows the incorporation of 3H-TdR in allogeneic T cells activated by the regulatory dendritic cells of the present invention (NK-DC), untreated dendritic cells (CTR-DC), and immature dendritic cells (unstim-DC). Black circles indicate NK-DC, white circles indicate CTR-DC, and × indicates unstim-DC.
[Figure 2] Fig. 2 shows the survival rate of heart transplant C3He/J mice (into which the regulatory dendritic cells of the present invention (NK-DC) or untreated dendritic cells (CTR-DC) had been transferred C3He/J mice received of C57BL/6 heart grafts in the heart transplantation model.. A continuous line indicates NK-DC and dotted lines indicate CTR-DC or unstim-DC.

### Embodiments for Carrying Out the Invention

In the present invention, regulatory dendritic cells can be prepared by treating cells that can be induced to result in regulatory dendritic cells (e.g., dendritic cells or precursor cells thereof) in a test tube with a [1,2,4]triazolo[1,5-a]pyrimidine derivative, and, if desired, cytokines and/or inflammatory stimulants. Specifically, regulatory dendritic cells can be obtained by treating monocytes collected from peripheral blood, bone marrow, or the like, with differentiation factors (e.g. GM-CSF, IL-4), maturation factors (e.g. inflammatory cytokine, TNF-α and inflammatory substance, LPS), and the [1,2,4]triazolo[1,5-a]pyrimidine derivative. The order of treating with these cytokines and/or inflammatory substances, and the [1,2,4]triazolo[1,5-a]pyrimidine derivative is not particularly limited. Regulatory dendritic cells can be prepared by performing stimulation in an arbitrary order. For example, monocytes are *cultured in vitro* in the presence of GM-CSF and IL-4, so that the monocytes are caused to differentiate into dendritic cells, and then regulatory dendritic cells can be induced from the thus obtained dendritic cells using the [1,2,4]triazolo[1,5-a]pyrimidine derivative. At such time, monocytes may be first stimulated with GM-CSF and IL-4 to differentiate into dendritic cells and then stimulated with the [1,2,4]triazolo[1,5-a]pyrimidine derivative, or monocytes may be stimulated simultaneously with GM-CSF, IL-4, and the [1,2,4]triazolo[1,5-a]pyrimidine derivative. At this time, cytokines for differentiation to regulatory dendritic cells (e.g., IL-10 and TGF-β) may coexist with the [1,2,4]triazolo[1,5-a]pyrimidine derivative. Furthermore, mature regulatory dendritic cells can be obtained by giving inflammatory stimulation using TNF-α, LPS, or the like.

Cytokines or inflammatory stimulants to be used for preparing the regulatory dendritic cells of the present invention are not particularly limited. Examples of cytokines include cytokines having a function of inducing the differentiation of dendritic cells, such as GM-CSF, IL-4, IL-10, and TGF-β. Examples of inflammatory stimulants include lipopolysaccharides represented by LPS and cytokines such as TNF-α providing inflammatory stimulation.

Dendritic cells can be obtained by culturing monocytes in the presence af GM-CSF and/or IL-4 as described above. Monocytes in this case may be derived from peripheral blood, bone marrow, spleen cells, or umbilical cord blood. Furthermore, dendritic cells can also be isolated from these tissues and organs by a cell sorter or the like using as an indicator the expression of a surface antigen (e.g., CD1a) specific to the dendritic cells. A specific cell population can be isolated using a cell sorter according to a known method. Monocytes which are used for obtaining the regulatory dendritic cells of the present invention may be derived from a mammal such as primates, Rodentia, Carnivora, Artiodactyla, Perissodactyla and the like. Specific examples thereof include humans, monkeys, mice, rats, rabbits, cats, cattle, dogs, horses, and goats. The most preferable example thereof is a human. Among the above examples, preferably monocytes are derived from an animal of the same species as that of an animal to be subjected to prevention or treatment of disease using the regulatory dendritic cells of the present invention.

Monocytes and dendritic cells can be cultured by known culture techniques for lymphoid cells. As culture medium, RPMI1640 or DMEM can be used, for example. Cells can be cultured in these basal medium supplemented with appropriate antibiotics, animal serum, or the like. Culture vessels are also not limited Commercially available plates, dishes, and flasks can be appropriately selected and used depending on the culture scale.

A [1,2,4]triazolo[1,5-a]pyrimidine derivative to be used in the present invention is a compound disclosed in JP Patent Publication (Kokai) No. 2005-154335 A and International Patent Publication 2004/108729. Specifically, the [1,2,4]triazolo[1,5-a]pyrimidine derivative to be used in the present invention is preferably a compound represented by the following formula (1) or a pharmaceutically acceptable salt thereof.

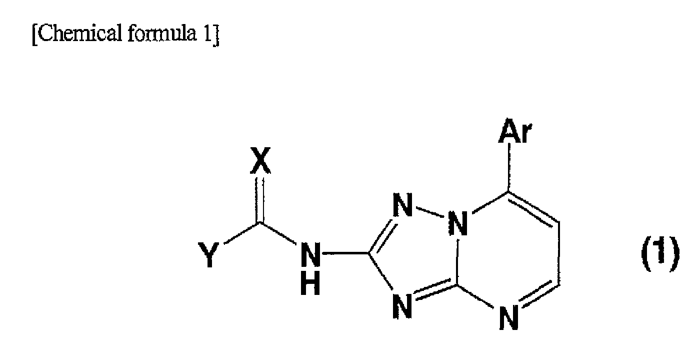

[wherein, Ar represents an aromatic hydrocarbon group or an aromatic heterocyclic group containing 1 to 4 heteroatoms, which may optionally have a substituent, X represents O, S, NH, N-CH₃, or N-CN, Y denotes a phenyl group that may optionally have a substituent, or an RNH group, and R represents a hydrogen atom, a cyano group, a linear, branched or cyclic alkyl group that may optionally have a substituent, an aromatic hydrocarbon group that may optionally have a substituent, or a 5- to 7-membered heterocyclic group that may optionally have a substituent and contains 1 to 4 heteroatoms independently selected from N, O, and S].

Specific example is [1,2,4]triazolo[1,5-a]pyrimidine-2-il urea derivative or a pharmaceutically acceptable salt thereof, wherein Ar is a phenyl group that may have a substituent or a 5- to 6-membered aromatic heterocyclic group that may have a substituent and contains 1 heteroatom selected from N, O, and S, wherein the phenyl group or the aromatic heterocyclic group may be substituted with 1 or 2 identical or different groups selected from the substituent group consisting of a halogeno group, a hydroxy group, a cyano group, a nitro group, a (C1-C6) alkyl group, a (C1-C6) alkoxyl group, and an alkylenedioxy group, X is O, and R is a phenyl group that may be optionally substituted with 1 to 3 identical or different groups selected from the substituent group consisting of a halogeno group, a hydroxy group, a (C1-C6) alkyl group, a (C1-C6) alkoxyl group, a (C1-C7) acyloxy group, a trifluoromethyl group and a trifluoromethoxy group or a group represented by general formula (2):

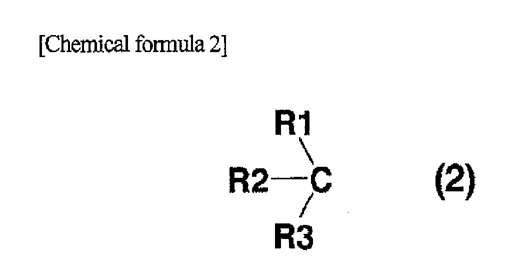

{wherein, R1 represents a hydrogen atom or a (C1-C6) alkyl group , R2 represents a hydrogen atom or a methyl group, R3 represents a hydrogen atom, a phenyl group (wherein the phenyl group may be optionally substituted with 1 group selected from the group consisting of a halogeno group, a hydroxy group, a (C1-C6) alkyl group and a (C1-C6) alkoxyl group), or a (C1-C10) alkyl group (wherein the alkyl group may be optionally substituted with 1 or 2 identical or different groups selected from the group consisting of a halogeno group, a hydroxy group, a (C1-C6) alkoxyl group, a (C1-C7) acyloxy group and a trifluoromethyl group)}.

Specific examples of the [1,2,4]triazolo[1,5-a]pyrimidine derivative to be used in the present invention include, but are not limited to, the following compounds:
(S)-1-(3-ethoxy-1-methylpropyl)-3-[7-(3,4-methylenedioxyphenyl)-[1,2,4]triazolo[1,5-a]pyrimidin-2-yl]urea;
(S)-1-(4-methoxy-1-methylbutyl)-3-[7-(4-methoxyphenyl-[1,2,4]triazolo[1,5-a]pyrimidin-2-yl]urea;
(S)-1-(4-hydroxy-1,4-dimethylpentyl)-3-[7-(4-methoxyphenyl)-[1,2,4]triazolo[1,5-a]pyrimidin-2-yl] urea;
(S)-1-(5-hydroxy-1,5-dimethylhexyl)-3-[7-(4-methoxyphenyl)-[1,2,4]triazolo[1,5-a]pyrimidin-2-yl] urea;
(S)-1-(5-hydroxy-1,5-dimethylhexyl)-3-{7-[3-(pyridin-3-ylmethoxy)phenyl]-[1,2,4]triazolo[1,5-a] pyrimidin-2-yl}urea;
(S)-1-(5-hydroxy-1,5-dimethylhexyl)-3-(7-thiophen-2-yl[1,2,4]triazolo[1,5-a]pyrimidin-2-yl)urea; (S)-1-[1-(3-methoxyphenyl)ethyl]-3-[7-(4-methoxyphenyl)-[1,2,4]triazolo[1,5-a]pyrimidin-2-yl]urea
(S)-1-[7-(3,4-methylenedioxyphenyl)-[1,2,4]triazolo[1,5-a]pyrimidin-2-yl]-3-[1-(3-methoxyphenyl) ethyl]urea;
(S)-1-[7-3,4-methylenedioxyphenyl)-[1,2,4]triazolo[1,5-a]pyrimidin-2-yl]-3-[1-(3,4,5-trimethoxyph enyl)ethyl]urea;
(S)-1-(7-thiophen-2-yl[1,2,4]triazolo[1,5-a]pyrimidin-2-yl)-3-[1-(3,4,5-trimethoxyphenyl)ethyl]urea;
(S)-1-[1-(3,4-methylenedioxyphenyl)ethyl]-3-{7-[3-(pyridine-3-ilmethoxy)phenyl]-[1,2,4]triazolo[1, 5-a]pyrimidin-2-yl}urea;
(S)-1-(1,5-dimethylhexyl)-3-[7-(4-hydroxyphenyl)-[1,2,4]triazolo[1,5-a]pyrimidin-2-yl]urea;
(S)-1-[7-(4-methoxyphenyl)-[1,2,4]triazolo[1,5-a]pyrimidin-2-yl]-3-(1-phenylethyl)urea;
4-chloro-2-methoxy-N-(7-thiophen-2-yl[1,2,4]triazolo[1,5-a]pyrimidin-2-yl)benzamide;
(S)-1-(1,5-dimethylhexyl)-3-[7-(4-methoxyphenyl)-[1,2,4]triazalo[1,5-a]pyrimidin-2-yl]urea;
1-(2-methoxyphenyl)-3-(7-thiophene-[1,2,4]triazolo[1,5-a]pyrimidin-2-yl)urea;
1-isopropyl-3-(7-thiophene-[1,2,4]triazolo[1,5-a]pyrimidin-2-yl)urea;
(R)-1-[7-(4-methoxyphenyl)-[1,2,4]triazolo[1,5-a]pyrimidin-2-yl]-3-(1-phenylethyl)urea; and
(R)-1-(5-hydroxy-1,5-dimethylhexyl)-3-[7-(4-methoxyphenyl)-[1,2,4]triazolo[1,5-a]pyrimidin-2-yl] urea

The most preferable specific examples of the [1,2,4]triazolo[1,5-a]pyrimidine derivative to be used in the present invention is (S)-(+)-1-(5-hydroxy-1,5-dimethylhexyl)-3-[7-(4-methoxyphenyl)-[1,2,4]triazolo[1,5-α]pyrimidin-2 -yl]urea.

According to the present invention, the use of the [1,2,4]triazolo[1,5-a]pyrimidine derivative for preparing regulatory dendritic cells and a reagent for inducing regulatory dendritic cells which comprises the [1,2,4]triazolo[1,5-a]pyrimidine derivative, are provided.

The concentration of the [1,2,4]triazolo[1,5-a]pyrimidine derivative to be used for culture ranges from 1 ng/mL to 5000 ng/mL, and preferably ranges from 10 ng/mL to 500 ng/mL. The concentration of GM-CSF, IL-4, IL-10, TGF-β, TNF-α, or LPS ranges from 1 ng/mL to 1000 ng/mL, and preferably ranges from 10 ng/mL to 100 ng/mL. Also, the number of days for culture which is required for inducing regulatory dendric cells, is not limited. For example, monocytes may be cultured together with the [1,2,4]triazolo[1,5-a]pyrimidine derivative, cytokines and/or inflammatory stimulation for about several days to 10 days. The expression of a surface antigen of monocytes or dendritic cells is examined by flow cytometry or the like, so that the culture duration required for inducing mature dendritic cells can be appropriately determined. Conditions such as concentrations and culture duration as to the [1,2,4]triazolo[1,5-a]pyrimidine derivative, cytokines and/or inflammatory substance to be used for inducing regulatory dendritic cells, can be determined by the extent of allogeneic T cell unresponsiveness or the phenotype of dendritic cells as an indicator.

When the regulatory dendritic cells of the present invention are used for treating disease, the regulatory dendritic cells of the present invention are incubated with antigens relating to the disease to be treated. As an antigen, in the case of autoimmune disease or allergic disease, an antigen protein or peptide existing in a tissue and/or an organ relating to such disease, or RNA or DNA encoding it, or a modified product thereof is used. In the case of graft rejection or graft-versus-host disease, donor- or a recipient-derived antigens are used.

Dendritic cells for treating autoimmune disease or allergic disease are incubated with the antigens for a period ranging from 10 days to 1 day including the final date for culture. In the case of protein antigens, they are added *in vitro* at a concentration ranging from 1 ng/ml to 10 mg/ml, and preferably ranging from 10 ng/ml to 5 mg/ml. When regulatory dendritic cells further stimulated with an inflammatory stimulant are used, in general, an antigen is added simultaneously with or before inflammatory stimulation.

The regulatory dendritic cells of the present invention can be used for therapeutic drugs, prophylactic drugs and the like for immune diseases and the like due to immunological abnormalities. Examples of immune disease include transplantation rejections, autoimmune disease, allergic disease, inflammatory disease, septicemia, and shock.

Examples of transplantation rejections include acute rejection, graft-versus-host disease, and chronic rejection upon transplantation. The regulatory dendritic cells of the present invention can be used for inducing immunologic tolerance, for example, in addition to the treatment and prevention of transplantation rejections. A transplant may be any organs such as bone marrow, kidney, liver, heart, and pancreas. Regarding donor-to-host relationship, transplantation can be heterologous transplantation, homologous transplantation, or ABO-incompatible transplantation, for example. Also, the regulatory dendritic cells of the present invention can be used for the purpose of immunosuppression and long-term graft survival upon transplantations such as bone marrow transplantation, peripheral blood stem cell transplantation, and umbilical cord blood stem cell transplantation that are performed for cancer treatment, autoimmune disease treatment, gene therapy, and regeneration medicine.

Examples of autoimmune disease include rheumatoid arthritis, multiple sclerosis, systemic lupus erythematosus, discoid lupus erythematodes, Sjogren's syndrome, Crohn's disease, ulcerative colitis, idiopathic thrombocythemia, aplastic anemia, autoimmunehepatitis, autoimmune myocarditis, idiopathic thrombocytopenic purpura, Behchet's disease, autoimmune gastritis, insulin dependent diabetes mellitus, myasthenia gravis, polymyositis, scleroderma, mixed connective tissue disease, ankylosing spondylitis, chronic thyroiditis, pemphigus, Guillain-Barre syndrome, and HTLV-1-associated myelopathy. Also, examples of allergic disease include atopic dermatological disease, pollinosis, contact hypersensitivity, asthma, psoriasis, and anaphylaxis. Examples of inflammatory disease include polyarteritis, sarcoidosis, glomerulonephritis, nephrotic syndrome, intractable angiitis, and Wegener's syndrome.

The dosage of the regulatory dendritic cells of the present invention is appropriately determined so that desired effects can be exerted on an administration subject. Specifically, about 0.5 x 10⁵ to 10⁹ of the regulatory dendritic cells of the present invention are administered to an administration subject (individual) intravenously, subcutaneously, or intradermally (and preferably intravenously). The medium to be used upon administration may be any generally-employed medium and is not particularly limited, as long as it causes no adverse effect or the like in an administration subject and does not cause deterioration of the functions of the regulatory dendritic cells of the present invention. Examples of such a medium include medium such as RPMI1640 and DMEM, Hank's buffer, phosphate buffered saline (PBS), a physiological saline solution, and a dextrose solution. Examples of an administration subject are not particularly limited as long as it is a mammal. Examples include primates, Rodentia, Carnivora, Artiodactyla, and Perissodactyla, such as a human, a monkey, a mouse, a rat, a rabbit, a cat, cattle, a dog, a horse, and a goat. A preferable example thereof is a human.

The timing for administration of the agents for preventing or treating various diseases to administration subjects is not particularly limited. Such an agent can be administered as needed. Particularly in the case of graft rejection or graft-versus-host disease associated with organ and/or tissue transplantation, administration is preferably performed before the procedure (transplantation) by which the onset of such a disease is predicted. The timing for administration and the dosage of human immunoregulatory dendritic cells can be appropriately determined depending on disease type, disease severity, patient conditions, and the like.

The present invention further encompasses a pharmaceutical composition or a pharmaceutical kit which comprises the regulatory dendritic cells of the present invention. The pharmaceutical composition of the present invention is not particularly limited, as long as it comprises the regulatory dendritic cells of the present invention. An example thereof is a pharmaceutical composition prepared by suspending the regulatory dendritic cells of the present invention in a medium that does not cause any adverse effect or the like in an administration subject as described above and does not deteriorate the functions of the regulatory dendritic cells of the present invention. A pharmaceutical composition prepared by adding a medicinally acceptable additive that maintains the state of the regulatory dendritic cells of the present invention or other agents is also included in the examples thereof The term "pharmaceutical kit of the present invention" refers to a combination of the regulatory dendritic cells of the present invention or the pharmaceutical composition of the present invention with (an)other agents. Examples of other agents are not limited, as long as they are known therapeutic drugs or prophylactic drugs for immune disease or the like resulting from immunological abnormalities, and include immunoregulatory drugs such as salazosulfapyridine, bucillamine, gold sodium thiomalate, D-penicillamine, auranofin, actarit, and lobenzarit, immunosuppressive drugs such as methotrexate, leflunomide, tacrolimus, mizoribine, cyclophosphamide, azathioprine, cyclosporine, and mycophenolate mofetil, biological preparations such as infliximab, etanercept, adalimumab, tocilizumab, and rituximab, NSAIDs such as mefenamic acid, diclofenac sodium, nabumetone, etodolac, sodium loxoprofen, and meloxicam, steroid drugs such as prednisolone, methylprednisolone, methylprednisoloneacetate(ester), sodium methylprednisolonesuccinate(ester), dexamethasone, betamethazone, and hydrocortisone, NRTI such as zidovudine, lamibudine, abacavir, and emtricitabine, a nucleotide-based reverse transcriptase inhibiting drug such as tenofovir, NNRTI such as efavirenz, protease inhibiting drugs such as atazanavir, fosamprenavir, lopinavir, ritonavir, and darunavir, and an integrase inhibiting drug such as raltegravir. In addition, regarding the pharmaceutical kit of the present invention, the order for administration of the regulatory dendritic cells of the present invention or the pharmaceutical composition of the present invention and other agents is not limited.

The regulatory dendritic cells of the present invention are explained in the following Examples by exemplifying several embodiments. However, the present invention is not limited to these Examples.

### Examples

### Collection of C57BL/6 mouse bone marrow cells

A thigh bone was excised from each C57BL/6 mouse (SLC, male, 10- to 12-week-old), both ends of the thigh bone were cut in RPMI1640 medium, and thus bone marrow tissue was removed from the interior of the bone. A single-cell suspension was prepared using a 26G syringe from the bone marrow tissue, so that C57BL/6 mouse bone marrow cells were obtained.

Stimulation of differentiation of C57BL/6 mouse bone marrow cells into dendritic cells and maturation of the resultant

The C57BL/6 mouse bone marrow cells were cultured for 7 days in 10% FCS-RPMI1640 medium containing 20 ng/mL GM-CSF (Peprotech, origin: rabbit) and 20 ng/mL IL-4 (Peprotech, origin: rabbit) so as to cause the cells to differentiate into dendritic cells. Subsequently, 20 ng/mL TNF-α (Peprotech, origin: rabbit) was added for maturation. Treatment with NK026680 was performed by adding 50 ng/mL NK026680 on days 2 and 4 of culture and adding 250 ng/mL NK026680 on day 6 of culture.

### Collection of C57BL/6 mouse spleen cells and T cells

The spleen was excised from each C57BL/6 mouse (SLC, male, 10- to 12-week-old), spleen cells were removed from the spleen in RPMI1640 medium, a single-cell suspension was prepared using a 26G syringe, and thus C57BL/6 mouse spleen cells were obtained. C57BL/6 mouse T cells were separated and collected from C57BL/6 mouse spleen cells using nylon wool (R&D systems).

### C57BL/6 mouse spleen cell extract

C57BL/6 mouse spleen cells were disrupted thoroughly using an ultrasonic disruptor and then frozen at -80°C. Subsequently, frozen (disrupted) cells were thawed and then centrifuged (300 g for 5 minutes), so as to remove cell fragments. The supernatant of the resultant was used as an extract.

### Collection of C3He/J mouse bone marrow cells

A thigh bone was excised from each C3He/J mouse (SLC, male, 10- to 12-week old), both ends of the thigh bone were cut in RPMI1640 medium, and then bone marrow tissue was removed from the interior of the bone. A single cell suspension was prepared from the bone marrow tissue using a 26G syringe, so that C3He/J mouse bone marrow cells were obtained.

### Differentiation of C3He/J mouse bone marrow cells into dendritic cells

The C3He/J mouse bone marrow cells were cultured for 7 days in 10% FCS-RPMI1640 medium containing 20 ng/mL GM-CSF and 20 ng/mL IL-4 so as to cause the cells to differentiate into dendritic cells. Treatment with NK026680 was performed by adding 50 ng/mL NK026680 on days 2 and 4 of culture and adding 250 ng/mL NK026680 on day 6 of culture. Also, on day 6, the C57BL/6 mouse spleen cell extract was also added.

### BALB/c mouse spleen cell extract

The spleen was excised from each BALB/c mouse (SLC, male, 10- to 12-week-old), spleen cells were removed from the spleen in RPMI1640 medium, a single-cell suspension was prepared using a 26G syringe, and thus BALB/c mouse spleen cells were obtained. The spleen cells were disrupted thoroughly using a ultrasonic disruptor and then frozen at -80°C. Subsequently, frozen (disrupted) cells were thawed and then centrifuged (300 g for 5 minutes), so as to remove cell fragments. The supernatant of the resultant was used as an extract.

### (Example 1)

The BALB/c mouse-derived spleen cell extract was added to dendritic cells derived from bone marrow cells which were collected from the C57BL/6 mouse, thereby causing cells to present a BALB/c antigen. The effect of incorporating the BALB/c antigen was confirmed by measuring the expression of I-Ad (antibody: BD Pharmingen), which is a BALB/c major histocompatibility complex (MHC), by flow cytometry. At this time, cells were cultured in the presence or the absence of NK026680, and the cells were allowed to mature with TNF-α (Peprotech, origin: rabbit). Maturation of dendritic cells was confirmed by flow cytometric analysis of the expression of the mature dendritic cell marker. NK026680-treated dendritic cells (NK-DC) of the present invention and untreated dendritic cells (CTR-DC) were incubated with fluorescence-labeled antibodies against CD40, CD80 and CD86. Mean fluorescence intensity (MFI) was measured by flow cytometry, so that the expression levels of CD40, CD80, and CD86 were compared.

Results of Example 1 are shown in Table 1. MFIs representing the binding amounts of fluorescence-labeled antibodies against CD40, CD80, and CD86 were compared As a result, the expression levels of CD40, CD80 and CD86 of dendritic cells treated with NK026680 of the present invention (NK-DC) were found to be lower than those of untreated dendritic cells (CTR-DC). The low-level expression of the costimulatory molecules is a property of regulatory dendritic cells, and it was demonstrated that the dendritic cells (treated with NK026680) of the present invention are regulatory dendritic cells.

**[Table 1]**

| | Treatment with NK026680 | No treatment |
|---|---|---|
| CD40 (MFI) | 159 | 244 |
| CD80 (MFI) | 320 | 659 |
| CD86 (MFI) | 217 | 356 |

### (Example 2)

In this Example 2, in a manner similar to that in Example 1, the dendritic cells of the present invention treated with NK026680 and untreated dendritic cells, which had been obtained by differentiation and maturation of C57BL/6 mouse bone marrow cells, were obtained. After stimulation with TNF-α, cells were cultured for 24 hours. The thus obtained culture supernatant was collected, and then the concentrations of immune-response-stimulating cytokines, IL-6 and IL-12p40, were measured by ELISA (BD Bioscience) (n = 3).

The results of Example 2 are shown in Table 2. The concentrations of IL-6 and IL-12p40 contained in the culture supernatant of dendritic cells treated with NK026680 were lower than those in the culture supernatant of untreated dendritic cells. The low-level production of immune-response-stimulating cytokines (IL-6 and IL-12p40) is a property of regulatory dendritic cells, and it was demonstrated that the dendritic cells of the present invention treated with NK026680 are regulatory dendritic cells.

**[Table 2]**

| | Treatment with NK026680 | No treatment |
|---|---|---|
| IL-6 (pg/mL) | 219 ± 94 | 1235 ± 272 |
| IL-12p40 (pg/mL) | 347 ± 100 | 1555 ± 650 |

### (Example 3)

In this Example 3, in a manner similar to that in Example 1, the regulatory dendritic cells of the present invention treated with NK026680 (NK-DC) and untreated dendritic cells (CTR-DC), which had been obtained by differentiation and maturation of C57BL/6 mouse bone marrow cells, were obtained. Also, C57BL/6 mouse bone marrow cells were caused to differentiate into dendritic cells and then caused to present the BALB/c antigen in a manner similar to that in Example 1. Immature dendritic cells (unstim-DC) not caused to mature using TNF-α were also obtained. C57BL/6 mouse T cells were mixed with each of the 3 types of dendritic cell, and then incorporation of 3H-TdR (GE HealthCare) was measured. The amount of 3H-TdR incorporated represents the capacity of dendritic cells to induce T cell activation against alloantigen.

The results of Example 3 are shown in Fig. 1. NK-DC was found to have lower capacity to induce T cell activation against the BALB/c antigen than CTR-DC. Also, NK-DC was found to have a capacity to induce T cell activation equivalent to that of unstim-DC. Specifically, it was demonstrated that the capacity of NK-DC to activate T cell induction was decreased to the same level as that of immature dendritic cells even when subjected to alloantigen stimulation.

### (Example 4)

In this Example 4, the extract of C57BL/6 mouse spleen cells was added to dendritic cells derived from C3He/J mouse bone marrow cells, so as to cause the cells to present a C57BL/6 antigen. The effect of incorporating the C57BL/6 antigen was confirmed by measuring the expression of I-Ab (antibody: BD Pharmingen), which is the MHC of BALB/c, by flow cytometry. At this time, the cells were cultured in the presence or the absence of NK026680. The dendritic cells (NK-DC) treated with NK026680 and the untreated dendritic cells (CTR-DC) were separately transferred to C3He/J mice (SLC, male, 10- to 12-week-old) by intravenous administration (number of cells administered: 3 x 14⁵ cells/mouse; and medium: phosphate buffered saline (PBS)). At this time, a group (n = 6) wherein no dendritic cells are transferred (no treatment) was also prepared. 7 days later, hearts of C57BL/6 mice (SLC, male, 10- to 12-week old) were transplanted into a group to which NK-DC had been transferred, a group to which CTR-DC had been transferred, and No-treat group, and then the survival rates of the 3 groups were compared.

The results of Example 4 are shown in Fig. 2. The group to which NK-DC had been transferred was observed to have significantly improved survival rate and prolonged survival period compared with the group to which CTR-DC had been transferred and the No-treat group.

### Industrial Applicability

According to the method for preparing regulatory dendritic cells of the present invention, a large amount of regulatory dendritic cells can be obtained even though the method is very convenient. Also, the regulatory dendritic cells which are prepared by the method for preparing regulatory dendritic cells of the present invention are useful for preventing and treating disease resulting from immunological abnormalities and excessive immunoresponses.

## Claims

1. A method for preparing regulatory dendritic cells, which comprises culturing cells that can be induced to result in regulatory dendritic cells in the presence of a [1,2,4]triazolo[1,5-a] pyrimidine derivative.

2. The method for preparing regulatory dendritic cells according to claim 1, wherein the [1,2,4]triazoio[1,5-a]pyrimidine derivative is (S)-(+)-1-(5-hydroxy-1,5-dimethylhexyl)-3-[7-(4-methoxyphenyl)-[1,2,4]triazolo[1,5-a]pyrimidine-2-il] urea.

3. The method for preparing regulatory dendritic cells according to claim 1 or 2, wherein the cells that can be induced to result in regulatory dendritic cells are dendritic cells or precursor cells thereof

4. The method for preparing regulatory dendritic cells according to claim 3, wherein the precursor cells of dendritic cells are monocytes.

5. The method for preparing regulatory dendritic cells according to any one of claims 1 to 4, wherein the cells that can be induced to result in regulatory dendritic cells are cells derived from peripheral blood, bone marrow, spleen, or umbilical cord blood.

6. The method for preparing regulatory dendritic cells according to any one of claims 1 to 5, which comprises culturing the cells in the presence of GM-CSF and IL-4.

7. The method for preparing regulatory dendritic cells according to any one of claims 1 to 6, which comprises culturing the cells in the presence of TNF-α and/or LPS.

8. A regulatory dendritic cell, which is prepared by the preparation method according to any one of claims 1 to 7.

9. The regulatory dendritic cell according to claim 8, wherein the expression levels of CD40, CD80, and CD86 are lower than those of cells obtained by culturing cells that can be induced to result in regulatory dendritic cells in the absence of a [1,2,4]triazolo[1,5-a]pyrimidine derivative.

10. The regulatory dendritic cell according to claim 8 or 9, wherein the production levels of IL-6 and IL-12p40 are lower than those of cells obtained by culturing cells that can be induced to result in regulatory dendritic cells in the absence of the [1,2,4]triazolo[1,5-a]pyrimidine derivative.

11. The regulatory dendritic cell according to any one of claims 8 to 10, which has a lower capacity to induce T cell activation against an alloantigen than cells obtained by culturing cells that can be induced to result in regulatory dendritic cells in the absence of the [1,2,4]triazolo[1,5-a] pyrimidine derivative.

12. A pharmaceutical composition or a pharmaceutical kit, which comprises the regulatory dendritic cell according to any one of claims 8 to 11.

13. The pharmaceutical composition or a pharmaceutical kit according to claim 12, which is used for preventing and/or treating transplantation rejections, graft-versus-host disease, autoimmune disease, allergic disease, inflammatory disease, septicemia or shock which results from abnormal responses and excessive responses of the immune system.

14. Use of a [1,2,4]triazolo[1,5-a]pyrimidine derivative for preparing regulatory dendritic cells.

15. The use according to claim 14, wherein the [1,2,4]triazolo[1,5-a]pyrimidine derivative is (S)-(+)-1-(5-hydroxy-1,5-dimethylhexyl)-3-[7-(4-methoxyphenyl)-[1,2,4]triazolo[1,5-a] pyrimidin-2-yl]urea.

16. A reagent for inducing regulatory dendritic cells, which comprises a [1,2,4]triazolo[1,5-a]pyrimidine derivative.

17. The reagent for inducing regulatory dendritic cells according to claim 16, wherein the [1,2,4]triazolo[1,5-a]pyrimidine derivative is (S)-(+)-1-(5-hydroxy-1,5-dimethylhexyl)-3-[7-(4-methoxyphenyl)-[1,2,4]triazolo[1,5-a]pyrimidin-2-yl]urea.
